# EUROPEAN PATENT APPLICATION

(11) **EP 2 383 331 A1**
(43) Date of publication of application: **02.11.2011**
(21) Application number: 09838804.4
(22) Date of filing: 26.01.2009
(51) Int. Cl.: C12M 1/00, C12M 1/28

(54) **METHOD OF PRODUCING CELL CULTURE SCAFFOLD**

(71) Applicant: Empire Technology Development LLC, Wilmington, DE 19808 (US)
(72) Inventor: KUSUURA, Takahisa, Kawasaki-shi Kanagawa 213-0013 (JP)
(74) Representative: Grünecker, Kinkeldey, Stockmair & Schwanhäusser
(86) International application number: PCT/JP2009/051166
(87) International publication number: WO 2010/084613

(57) **Abstract**

Problem to be Solved

To provide a simple method for producing a cell culture scaffold material having a nanoporous surface in which the pore density, pore diameter and pore diameter distribution are easily controlled.

Solution

A method for producing a cell culture scaffold material, comprising: providing a substrate; forming a layer comprising a material having a plurality of nanoparticles dispersed in a matrix on the substrate; and selectively removing the nanoparticles from the layer comprising a material having a plurality of nanoparticles dispersed in a matrix.

## Description

### Technical Field

This application relates to a method for producing a cell culture scaffold having a nanoporous surface or a surface having recesses of the nano order (specifically, having a pore diameter of 1,000 nm or less).

### Background Art

A product having a nanoporous surface has been proposed to be utilized as a cell culture scaffold material (a material serving as a scaffold in culturing cells) for research and for medical use in the fields of agriculture, regeneration medicine, and the like (see Patent Documents 1 and 2).

A fine pattern processing technology using electron beam exposure or X ray exposure is a potential method for producing a product having a nanoporous surface. As a method utilizing a naturally formed structure, there is a well known method using a nanoporous alumina anodized film formed when aluminium is anodized in an acidic electrolyte (see Patent Document 3).

Patent Document 1
   Japanese Patent Laid-Open No. 2001-157574
Patent Document 2
   Japanese Patent No. 4159103
Patent Document 3
   Japanese Patent Laid-Open No. 11-200090

### Disclosure of the Invention

### Problems to be Solved by the Invention

However, the fine pattern processing technology requires complicated treatment using exposure equipment such as an electron beam exposure device or an X ray exposure device. The nanoimprint technology adopted for forming a nanoporous surface in Patent Document 2 is that in which a mold having a relief structure of the nano order is subjected to pattern transcription to an object to be processed, and requires close condition supervision, including the shift of the mold in the transcription step. It is also difficult to render the area of the object large because of high cost of making the mold. Although not only a cell culture scaffold material having a planar form but also, for example, that having a three-dimensional contour mimicking a biological organ or tissue is especially required, it is difficult to apply fine pattern processing or nanoimprinting on a product having such a complicated three-dimensional contour.
In addition, for efficient culture, it is desired to suitably set the density (porosity), pore diameter, pore diameter distribution and the like of fine recesses on the surface of the cell scaffold material depending on the type and the like of the cell to be cultured; however, it is difficult to control the density, pore diameter and pore diameter distribution of the recesses for the method disclosed in Patent Document 1 and for the method using an alumina anodized film.
Thus, a simple method is required for producing a cell culture scaffold material having a nanoporous surface in which the density, pore diameter and pore diameter distribution of the recesses can be controlled within a desired range.

### Means for Solving the Problems

As a result of intensive studies on a method for forming a nanoporous surface, the present inventor has found that providing a material with dispersed nanoparticles in a matrix and selectively removing the nanoparticles therefrom result in the formation of recesses of the nano order as removal traces thereof on the surface of the material. Then, this finding has been applied to the production of a cell scaffold material, thereby accomplishing the present embodiment.

### Best Mode for Carrying Out the Invention

The present embodiment is described below; however, the present invention is not intended to be limited thereto.
According to the present embodiment, a layer comprising a material having a plurality of nanoparticles dispersed in a matrix is formed on a substrate, followed by selectively removing the nanoparticles therefrom to render the surface a nanoporous surface. The method for selectively removing the nanoparticles is not limited; for example, the nanoparticles may be selectively removed by immersing the layer comprising a material having a plurality of nanoparticles dispersed in a matrix in a liquid capable of dissolving the nanoparticles but not dissolving the matrix to selectively release only the nanoparticles into the liquid. The nanoparticles may also be selectively removed by firing the particles at a temperature enabling the nanoparticles to burn but not allowing the matrix to burn.

The material constituting the matrix is not limited and may be selected as needed depending on the type of cells to be cultured, the environment of usage, and the like; examples thereof can include thermoplastic resins, hardening resins, elastomers, and celluloses.
Specific examples of the thermoplastic resins include polyesters; polyolefins; polyamides; polycarbonates; polyimides; polystyrenes or styrene copolymers; and fluororesins (polymers each obtained by polymerizing a monomer containing fluorine in the molecule) such as polytetrafluoroethylene (PTFE), tetrafluoroethylene-perfluoroalkoxyethylene copolymer (PFA), tetrafluoroethylene-hexafluoropropylene copolymer (FEP), tetrafluoroethylene-ethylene copolymer (ETFE), polychlorotrifluoroethylene (PCTEF), chlorotrifluoroethylene-ethylene copolymer (ECTEF), polyvinylidene fluoride, and polyvinyl fluoride. Specific examples of the hardening resins include epoxy resins, phenol resins, acrylic resins, and urethane resins. Specific examples of the elastomers include natural rubber, styrene-butadiene copolymer and hydrogenated products thereof.
When the cell culture scaffold material is used by being embedded in a living body, a biodegradable material or a material capable of being absorbed or degraded in a living body can be used as the material constituting the matrix. Specific examples of the biodegradable material include, although partly overlapping with the above, polylactic acid, polycaprolactone, polyglycolic acid, polybutylene carbonate, gelatin, chitin, collagen, chitosan, keratin, apatite, polyamino acid, hyaluronic acid, and polysaccharides.
In addition, oxides such as quartz and aluminium oxide; nitrides; glass; other various ceramics; and metals such as Au, Pt, and Si can be also used.

The material constructing the nanoparticle is not limited and may be selected as needed considering a balance with the material making up the matrix.
For example, when the selective removal of the nanoparticles is performed by immersion in a liquid capable of dissolving the particles but not dissolving the matrix, any material may be adopted as far as the liquid capable of dissolving the particles but not dissolving the matrix can be obtained.
For example, when a thermoplastic resin, a hardening resin, an elastomer, a ceramic, a metal (e.g., Au or Pt), or the like is used as the matrix, a metal particle such as Ag, Cu, Fe, Ni, Cr, and Zn particles may be employed as the nanoparticle. In addition, when a ceramic, a metal, or the like is used as the matrix, particles comprising a polymer material capable of being dissolved in an organic solvent may be employed as the nanoparticle.

The diameter and the diameter distribution of nanoparticles are not limited. The pore diameter of recesses formed after the release of the nanoparticles is almost equal to the diameter of the nanoparticles; thus, the diameter and the diameter distribution of the nanoparticles can be adjusted to control the pore diameter and the pore diameter distribution on the nanoporous surface. It may be set, for example, to 1,000 nm to 100 nm, to 100 nm to 10 nm, or to 10 nm to 1 nm depending on the type and the like of cells to be cultured.
As used herein, the "particle diameter" refers to the biaxial average diameter or the average value of the minor and major diameters when the particle is observed two-dimensionally under a transmission electron microscope (TEM) or the like. Here, the minor and major diameters are such short and long sides, respectively, of a circumscribed rectangle that the area obtained when the rectangle is circumscribed to the particle is minimal. Then, the average particle diameter refers to the average of the diameters of 100 particles randomly selected in an identical field of vision in subjecting the particles to two-dimensional observation. In addition, as used herein, the "pore diameter" refers to the diameter of pores determined by a mercury intrusion technique according to JIS R1655.

Likewise, the shape of the nanoparticle is not limited; nanoparticles having a desired shape of recesses on the nanoporous surface may be used depending on the type of cells to be cultured, the environment of usage, and the like. The nanoparticles may be produced by any method.

The content of nanoparticles in the matrix is not limited, and may be determined as needed depending on the desired density of recesses. Release of the nanoparticle into the immersion liquid requires the exposure of at least a portion of the nanoparticle from the matrix.
From such a viewpoint, depending on the desired density of recesses, the content of the nanoparticles may be 30% by volume or more, 50% by volume or more, 70% by volume or more, or 90% by volume or more based on the total volume of the nanoparticles and the material constituting the matrix.

The shape and the material of the substrate are not limited, and may be suitably selected depending on the application.
For example, the shape of the substrate may be a planar form or a three-dimensional contour. Specifically, a substrate may also be used which has a complicated three-dimensional contour mimicking a biological organ or tissue such as a body part or blood vessel, or the like.
A substrate may also be used which has the form of a cell culture vessel such as a petri dish or a multi-well plate.
The material of the substrate used may be not only that having rigidity but also that having flexibility such as fabric or non-woven paper. Specifically, those exemplified as the materials of the matrix may be used. Particularly when the cell culture scaffold material is used by being embedded in a living body, a biodegradable material may also be employed.

The method for forming a layer comprising a material having a plurality of nanoparticles dispersed in the matrix on the substrate is not limited; any method may be adopted. For example, (co-)metallizing such as vacuum metallizing or ion plating may also be utilized.

According to one embodiment, a layer comprising a material having nanoparticles dispersed in the matrix can be formed by providing a nanoparticle-dispersed liquid containing a material constituting the matrix and coating the liquid on the substrate.
In the nanoparticle-dispersed liquid containing a material constituting the matrix, the material constituting the matrix may be dissolved or dispersed in the nanoparticle-dispersed liquid. Specific examples of the nanoparticle-dispersed liquid containing a material constituting the matrix include a liquid in which the nanoparticles are dispersed in a solution having a material constituting the matrix dissolved, and a liquid obtained by dispersing both of the particles comprising a material constituting the matrix and the nanoparticles in a dispersion medium. For example, the nanoparticle-dispersed liquid may be prepared by dissolving the material constituting the matrix in water or an organic solvent such as an alcohol, a ketone solvent, an ester solvent, a hydrocarbon solvent, or a halogen-containing hydrocarbon solvent and then dispersing the nanoparticles in the solution, or the dispersed liquid may be prepared by dispersing the particles comprising a material constituting the matrix in a dispersion medium such as water and then dispersing the nanoparticles in the dispersed liquid, or vice versa.

The dispersibility of the nanoparticles in the dispersed liquid may be improved by modifying the surface thereof so that the release of the nanoparticles in the subsequent step is not prevented. Examples of the surface-modified nanoparticle include a nanoparticle whose surface is coated with a protein or peptide or a low molecular weight vinyl pyrrolidone.
The surface modification in which a protein or peptide is immobilized on the surface of nanoparticles can be carried out according to the method disclosed in Japanese Patent Laid-Open No. 2007-217331. Specifically, the nanoparticles are dispersed in water using a surfactant, and the protein or peptide is added to the resultant dispersion and the mixture was irradiated with an ultrasonic wave at pH 5.0 or more to replace the surfactant on the surface of the nanoparticles with the protein or peptide. As a result, an aqueous dispersion of the nanoparticles on the surface of which the protein or peptide is immobilized is obtained. For example, particles comprising a material constituting the matrix can be further dispersed in the aqueous dispersion of nanoparticles thus obtained to make a nanoparticle-dispersed liquid containing the material constituting the matrix.
The surface modification in which nanoparticles are coated with low molecular weight vinyl pyrrolidone can be carried out according to the method disclosed in Japanese Patent Laid-Open No. 2008-121043. Specifically, nano metal particles are prepared in the presence of low molecular weight vinyl pyrrolidone to provide low molecular vinyl pyrrolidone-coated nano metal particles. The low molecular vinyl pyrrolidone-coated nano metal particles thus obtained are dispersed, for example, in an organic solvent such as 1, 2-ethanediol. In the nanoparticle-dispersed liquid thus obtained, further, a material constituting the matrix can be dissolved, or particles comprising the material constituting the matrix can be dispersed to make a nanoparticle-dispersed liquid containing the material constituting the matrix.

The method for coating the nanoparticle-dispersed liquid on a substrate is not limited; for example, a heretofore known coating method such as spraying, spin coating or dip coating may be adopted.
After coating, the dispersion medium solvent is removed from the coated layer by drying or the like to form a layer comprising a material having a plurality of nanoparticles dispersed in the matrix. If necessary, the coated layer may be heated to sinter or melt the material constituting the matrix to change it into a firm continuous phase. When the material constituting the matrix is a polymer material, it can be heated at its glass transition temperature or higher.

According to another embodiment, the so-called mechanical alloying may be utilized. The mechanical alloying is a solid mixing method which involves mixing more than one type of solid while adding considerable energy thereto to repeatedly provoke the lamination, folding and rolling of the solids for fine mixing. In theory, the mixing can also be performed at an atomic level. According to the mechanical alloying, nanoparticles can be relatively easily and uniformly dispersed in the matrix.
The mechanical alloying is a method generally used in mixing metals. However, the present inventor has found that this method can also be applied to, for example, the mixing between polymer materials and between a polymer material and a metal or the like as far as they are materials capable of being folded and rolled.

Specifically, particles (powder) comprising a material constituting the matrix and particles (powder) comprising a material constructing the nanoparticle are provided, and mixed while adding considerable energy thereto.
Then, the solid mixture obtained by the mechanical alloying is melt coated directly on a substrate, or the solid mixture is dispersed in a suitable solvent and the resultant dispersion is coated on a substrate to form on the substrate a layer comprising a material having a plurality of nanoparticles dispersed in the matrix. As a method for coating the dispersion, the above-described method can be adopted. After coating, if necessary, the coated layer may be heated to sinter or melt the material constituting the matrix to change it into a firm continuous phase.

According to the mechanical alloying, a material having nanoparticles dispersed in the matrix can be formed without initially providing particles of the nano order because solid materials are folded and divided during the mixing. Thus, the diameter of those particles (powder) comprising a material constructing the nanoparticle provided in performing the mechanical alloying needs not be of the nano order, and may be, for example, 1 to 1,000 µm or 1 to 100 µm. The diameter of the particles (powder) comprising a material constituting the matrix is also not limited, and may be comparable to the diameter of the particles (powder) comprising a material constructing the nanoparticle or larger than that of the particles (powder) comprising a material constructing the nanoparticle.

According to the mechanical alloying, the mixing of metals can be carried out using the same as a heretofore known technique and apparatus. For example, the mechanical alloying can be performed under the mixing using a ball mill such as a rolling ball mill, a vibration mill or a planetary ball mill. Here, the collision energy of balls folds and rolls more than one type of solid particle.

The nanoparticles are selectively removed from that layer comprising a material having nanoparticles dispersed in the nanoparticle matrix formed as described above.
When the method involving immersion in a liquid capable of dissolving the nanoparticles but not the matrix to release the nanoparticles into the liquid is used as a method for selectively removing the nanoparticles, the liquid capable of dissolving the nanoparticles but not the matrix is not limited; a suitable liquid may be selected depending on the combination of the nanoparticle and the material constituting the matrix. For example, the following may be used: an acid solution such as hydrochloric acid, nitric acid or sulfuric acid; an alkali solution such as a sodium hydroxide aqueous solution or a potassium hydroxide aqueous solution; or an organic solvent.
The immersion time is not limited, and may be that sufficient to release the nanoparticles. During the immersion, supplementary treatment for promoting the release may be performed which includes irradiating the preparation with an ultrasonic wave.
After release, washing with water is carried out, if necessary, followed by drying to provide a cell culture scaffold material having a nanoporous surface.

### Industrial Applicability

The cell culture scaffold material having a nanoporous surface can be used in vivo or ex vivo as a scaffold for cell culture or tissue regeneration for the purpose of research, diagnosis, medical care, or the like.

## Claims

1. A method for producing a cell culture scaffold material, comprising:
providing a substrate;
forming a layer comprising a material having a plurality of nanoparticles dispersed in a matrix on the substrate; and
selectively removing the nanoparticles from the layer.

2. The method for producing a cell culture scaffold material according to claim 1, wherein the selective removal of the nanoparticles is carried out by immersing the layer comprising a material having a plurality of nanoparticles dispersed in a matrix in a liquid capable of dissolving the nanoparticles but not dissolving the matrix.

3. The method for producing a cell culture scaffold material according to claim 2, wherein the liquid capable of dissolving the nanoparticles but not dissolving the matrix is an alkali solution or an acid solution.

4. The method for producing a cell culture scaffold material according to claim 1, wherein the nanoparticle is a metal particle.

5. The method for producing a cell culture scaffold material according to claim 1, wherein the nanoparticle is an Ag particle.

6. The method for producing a cell culture scaffold material according to claim 1, wherein the matrix comprises a biodegradable material.

7. The method for producing a cell culture scaffold material according to claim 1, wherein the formation of a layer comprising a material having a plurality of nanoparticles dispersed in a matrix comprises:
providing a nanoparticle-dispersed liquid containing a material constituting the matrix; and
coating the nanoparticle-dispersed liquid on the substrate.

8. The method for producing a cell culture scaffold material according to claim 1, wherein the formation of a layer comprising a material having a plurality of nanoparticles dispersed in a matrix comprises:
mixing particles comprising a material constituting the matrix and particles comprising a material constructing the nanoparticle to provide a solid mixture; and
coating the solid mixture on the substrate.

9. The method for producing a cell culture scaffold material according to claim 8, wherein the mixing is carried out using a ball mill.

10. The method for producing a cell culture scaffold material according to claim 1, wherein the substrate has a three-dimensional contour.

11. A cell culture scaffold material produced by:
providing a substrate;
forming a layer comprising a material having a plurality of nanoparticles dispersed in a matrix on the substrate; and
selectively removing the nanoparticles from the layer.
